# FASCICULE DE BREVET EUROPEEN

(11) **EP 2 211 713 B1**
(45) Date de publication et mention de la délivrance du brevet: **31.07.2013**
(21) Numéro de dépôt: 08853946.5
(22) Date de dépôt: 06.11.2008
(51) Int. Cl.: A61B 5/0488, A61B 8/00

(54) **DISPOSITIF ET METHODE DE REPERAGE NON INVASIF D'UNE STRUCTURE TEL QU'UN NERF**
NICHTINVASIVE VORRICHTUNG UND VERFAHREN ZUR LOKALISIERUNG EINER STRUKTUR WIE Z. B. EINES NERVS
NON-INVASIVE DEVICE AND METHOD FOR LOCATING A STRUCTURE SUCH AS A NERVE

(30) Priorité: 08.11.2007 FR 0758884
(43) Date de publication de la demande: 04.08.2010
(60) Demande divisionnaire: 12190327.2
(73) Titulaire: Theraclion, 75014 Paris (FR)
(72) Inventeur: LACOSTE, François, 94250 Gentilly (FR)
(74) Mandataire: Hepp Wenger Ryffel AG
(86) Numéro de dépôt international: PCT/FR2008/052008
(87) Numéro de publication internationale: WO 2009/068793

(56) Documents cités:
- WO-A-2006/129046
- US-A1- 2005 240 126
- US-A1- 2006 085 049

## Description

La présente invention concerne le repérage non invasif de structures, tels que des nerfs, dans une zone d'un corps. Le repérage peut être une étape préalable à un traitement de tissus voisins de la structure.

Il est souvent nécessaire en médecine de repérer les nerfs, en particulier les nerfs moteurs, pour par exemple exciter un muscle ou encore éviter de détruire le nerf lors du traitement de tissus voisins.

Généralement on connaît la position approximative des nerfs par les atlas anatomiques, mais il peut être important de connaître leur position exacte dans une zone du corps, par exemple quand on veut effectuer des actes thérapeutiques dans son voisinage immédiat, sachant qu'on désire généralement le préserver. Le geste thérapeutique peut être une dissection ou consister à appliquer de l'énergie, par exemple ultrasonore, lumineuse (laser), radiofréquence ou encore micro-ondes ou radioactive. Connaissant sa position, on évitera de diriger le geste thérapeutique sur ce nerf ou juste à côté afin de le préserver.

Dans le cas par exemple du traitement des thyroïdes et des parathyroïdes, il est particulièrement important de préserver les nerfs laryngés récurrents, qui contrôlent les cordes vocales. Un autre exemple concerne les ablations de la prostate où il est important de préserver les nerfs érecteurs.

Il est aussi intéressant de repérer des nerfs sensitifs pour par exemple comprendre l'origine d'une douleur, ou les détruire afin de supprimer une douleur.

Le repérage précis des nerfs pose souvent problème, particulièrement lorsqu'il doit être effectué de façon non invasive, hors d'un contexte chirurgical. Les médecins utilisent l'imagerie (échographie, IRM, RX, scanner) pour repérer les structures anatomiques mais les nerfs ne se voient en général pas avec ces appareils en raison de leur petite taille ou d'un contraste insuffisant. Or de nombreuses méthodes thérapeutiques modernes sont « non invasives », c'est-à-dire non chirurgicales ; le geste est alors uniquement guidé par l'imagerie.

En général, si l'acte thérapeutique consiste en une chirurgie, le chirurgien peut voir les nerfs grâce à une dissection prudente et soignée. En cas de doute, il peut aussi utiliser un Moniteur d'intégrité nerveuse tel que le NIM-Response®, Nerve Integrity Monitoring System, commercialisé par la société Xomed. Cet appareil comporte des électrodes que le chirurgien place à proximité des nerfs à repérer (par exemple les nerfs laryngés récurrents) et une sonde trachéale, qui est installée dans la trachée du patient pendant les traitements. Un courant d'excitation émis par les électrodes parcourt le tissu puis le nerf ; les mouvements des muscles ou l'excitation électrique qu'ils reçoivent sont captés par la sonde trachéale. D'autres stimulateurs sont commercialisés, tels que The Silverstein™ Facial Nerve Monitor/Stimulator, Model S8n de la société WR Medical Electronics Co. On peut aussi détecter le mouvement des cordes vocales par endoscopie.

Ces techniques permettent ainsi de détecter des altérations éventuelles de ces nerfs pendant le geste thérapeutique. Ceci a été décrit dans la demande EP 1 409 079.

Cependant lorsque le traitement est effectué à distance de la cible, par exemple par des ultrasons focalisés (HIFU) ou au moyen d'un laser interstitiel ou d'une aiguille radiofréquence (RF), on n'a pas d'accès direct au nerf et on cherche donc un dispositif et une méthode non invasifs de repérage pour localiser avec précision ces nerfs, ou plus généralement des structures.

Pour atteindre ce but, la présente invention propose un dispositif de repérage non invasif d'une structure, telle qu'un nerf, dans une zone d'un corps ayant une surface externe, le dispositif comprenant un transducteur ultrasonore focalisé, avantageusement du type HIFU, adapté à produire un faisceau d'ultrasons à travers la surface externe dans la zone, le faisceau ayant un point focal capable d'être positionné sur la structure, des moyens de monitoring pour détecter une réponse de la structure lorsque soumise à une stimulation, et des moyens de stimulation pour stimuler la structure qui délivre une réponse détectée par les moyens de monitoring, caractérisé en ce que le dispositif comprend un appareil d'imagerie couplé mécaniquement au transducteur ultrasonore de sorte que l'appareil d'imagerie suit le point focal du transducteur pour visualiser sur une image la position du point focal du faisceau d'ultrasons dans la zone de corps, des moyens de marquage pour marquer la position de la structure sur l'image produite par l'appareil d'imagerie.

Selon l'invention, les moyens de stimulation comprennent au moins une électrode capable de délivrer une impulsion électrique à ladite structure.

Un principe de l'invention consiste donc à insonifier la région d'intérêt (par exemple celle dans laquelle on cherche un nerf) par un fin faisceau d'ultrasons, par exemple des ultrasons focalisés. On balaye le faisceau ultrasonore dans la zone de recherche et à chaque position du faisceau on émet des impulsions ultrasonores et on constate les effets de cette émission sur la transmission de l'influx nerveux. Lorsque qu'on obtient l'effet maximal, on sait que le nerf est situé dans la région du faisceau de maximale intensité. L'émetteur d'ultrasons est couplé à un appareil d'imagerie médicale (échographe, IRM, RX, scanner), de telle manière que la position du faisceau acoustique est connue par rapport à l'anatomie du patient. On peut ainsi repérer le nerf et marquer sa position sur l'image produite par l'appareil d'imagerie. L'énergie du faisceau acoustique est faible, de façon à ne pas détruire les nerfs ou les tissus dans la zone de recherche.

Selon l'invention les ultrasons peuvent être émis suivant une séquence de pulses de longueurs variables (code) afin d'améliorer le seuil de détection (Réduire la puissance d'US nécessaire) et filtrer les réponses parasites du system nerveux central.

Selon un autre aspect intéressant de l'invention, le dispositif de repérage comprend en outre des moyens de synchronisation pour synchroniser le faisceau d'ultrasons et la stimulation de manière à ce qu'ils parcourent ou traversent la structure en même temps ou avec un décalage temporel déterminé. Avantageusement, les moyens de synchronisation comprennent des moyens de décalage adaptés à générer un décalage dans le temps entre le faisceau d'ultrasons et la stimulation de sorte que le faisceau d'ultrasons arrive sur la structure avant la stimulation. On est ainsi certain que le faisceau d'ultrasons influe de manière optimale sur la réponse de la structure soumise à la stimulation. Lorsque la stimulation se présente sous la forme d'une impulsion électrique délivrée à un nerf, la durée de l'impulsion est très courte, il est donc préférable que le faisceau d'ultrasons parcoure le nerf légèrement avant que l'impulsion traverse le nerf. C'est pourquoi il est avantageux de prévoir un léger décalage entre le faisceau et la stimulation.

Selon un autre aspect avantageux de l'invention, ledit dispositif de repérage comprend des moyens de calcul pour déterminer la profondeur de la structure depuis la surface externe à partir du temps de vol acoustique du faisceau d'ultrasons dans la zone et la vitesse de propagation de la stimulation. En connaissant le temps de vol acoustique du faisceau d'ultrasons et la vitesse de propagation de la stimulation dans la structure, il est aisément possible de localiser la position axiale de la structure en profondeur, c'est-à-dire dans la direction de propagation acoustique.

Selon une autre caractéristique, les moyens de monitoring peuvent comprendre un appareil de monitoring de réponse nerveuse ou un appareil de monitoring de réponse musculaire, tel qu'un électromyographe, un endoscope ou un capteur de pression. Ainsi, il est possible de mesurer la réponse à la stimulation soit directement au niveau de la structure, par exemple un nerf, ou encore au niveau d'un organe qui dépend directement de la structure, comme par exemple un muscle.

La présente invention a également pour objet une méthode de repérage non invasive d'une structure, telle qu'un nerf, dans une zone d'un corps ayant une surface externe, caractérisée en ce qu'elle comprend d'insonifier la structure à partir de la surface externe, de faire un monitoring de la réponse de la structure à une stimulation et de visualiser la position de l'insonification de la structure. La méthode comprend de stimuler la structure avec des impulsions électriques. La méthode comprend de synchroniser l'insonification et la stimulation au niveau de la structure. La méthode peut également comprendre de déterminer la profondeur de la structure à partir de la surface extérieure en se servant du temps de vol acoustique du faisceau d'ultrasons dans la zone et de la vitesse de propagation de la stimulation.

Ainsi, la stimulation de la structure peut provenir d'une stimulation extérieure telle qu'une stimulation électrique appliquée à un nerf à l'aide d'une électrode d'excitation ou de stimulation. Il suffit alors de détecter directement la réponse de la stimulation au niveau d'un organe dépendant de la structure, comme par exemple un muscle. Dans ce dernier cas, il n'est pas nécessaire de prévoir des moyens de stimulation spécifiques, puisque le transducteur va remplir une double fonction de stimulation et de repérage. Le dispositif et la méthode de la présente invention peut ainsi se résumer à trois moyens ou étapes, à savoir envoyer un faisceau d'ultrasons sur une structure cible, détecter la réponse de la structure cible, et en cas de résultat positif, marquer ou enregistrer la position de la structure cible à l'aide d'un appareil d'imagerie couplé au transducteur.

L'invention sera maintenant plus amplement décrite en référence aux dessins joints donnant à titre d'exemple non limitatif un mode de réalisation de l'invention.

Sur les figures :
La figure 1 est une vue schématique d'un dispositif de repérage selon l'invention,
La figure 2 est également une vue schématique montrant une partie du dispositif de repérage de la figure 1 en place sur un patient, et
Les figures 3 et 4 sont des organigrammes pour comprendre les différentes étapes de la méthode de repérage de l'invention.

Pour des raisons de simplicité, il sera fait référence uniquement à un nerf dans la suite de la description comme exemple de structure cible à repérer à l'aide du dispositif et de la méthode de repérage non invasif de la présente invention. Bien entendu, d'autres types de structures que les nerfs peuvent être repérés grâce à la présente invention, que ces structures soient situées à l'intérieur d'un corps vivant ou d'un autre type de corps.

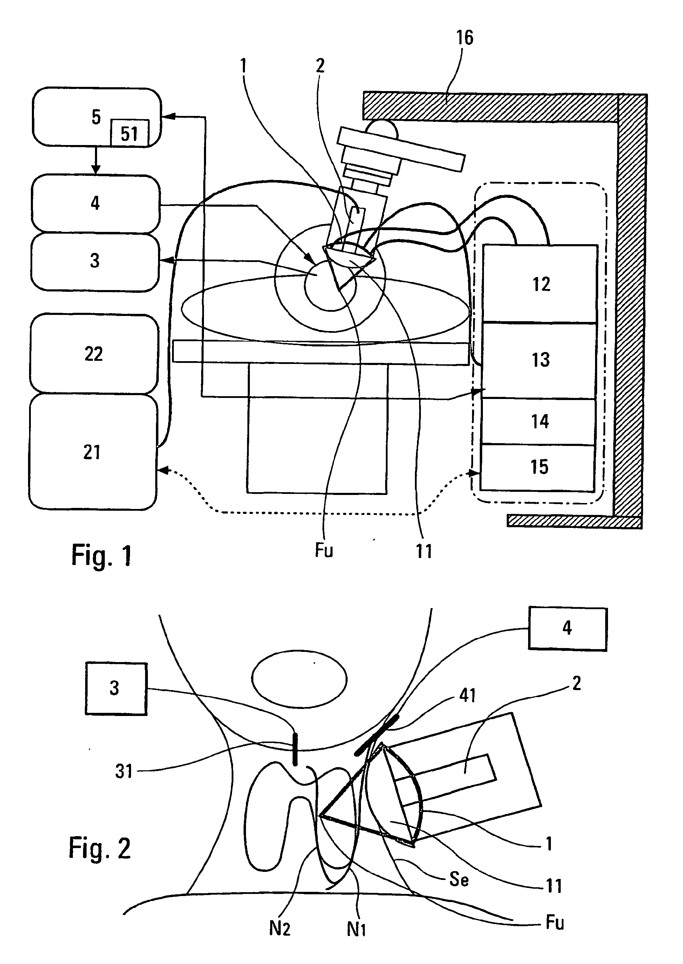

Il sera d'abord fait référence à la figure 1 pour décrire en détail les différents éléments constitutifs du dispositif de repérage de l'invention.

Le dispositif comprend tout d'abord une source d'émission acoustique 1 qui peut avantageusement être un transducteur ultrasonore adapté à produire un faisceau d'ultrasons Fu. De préférence, le transducteur ultrasonore est du type focalisé HIFU permettant de produire un faisceau focalisé d'ultrasons en un point focal précis. Le transducteur peut aussi être du type à barrettes. Le transducteur 1, comme on peut le voir sur la figure 2, peut comprendre une chambre remplie d'un fluide de couplage à travers lequel se propagent les faisceaux d'ultrasons. La chambre peut par exemple être délimitée par un ballonnet souple destiné à venir en contact intime avec une surface externe Se d'une zone d'un corps où se situe le nerf à rechercher. En général, la surface externe Se est la peau du patient. Pour faire circuler le liquide de couplage à l'intérieur de la chambre 11, il est en général prévu des moyens de circulation 12 qui permettent de réguler le débit et la température du fluide de couplage à l'intérieur de la chambre 11. Le transducteur pour fonctionner a bien entendu besoin d'une alimentation de puissance 13 ainsi que d'une commande de déplacement 14 qui permet de déplacer et de localiser avec précision le transducteur par rapport au patient. Pour ce faire, le transducteur 1 est de préférence monté sur un bras articulé 16. Enfin, le transducteur est couplé à un ordinateur 15 qui permet de gérer tous les paramètres du transducteur, comme sa puissance, sa fréquence, sa durée d'impulsion, etc.

Le dispositif de repérage de l'invention comprend également des moyens d'imagerie qui peuvent par exemple se présenter sous la forme d'une sonde échographique 2 couplée à un échographe 21 et un écran de visualisation 22. La sonde 2 est couplée mécaniquement au transducteur 1 comme on peut le voir sur les figures 1 et 2. Plus précisément, la sonde 2 et le transducteur 1 sont solidaires l'un de l'autre de sorte que la sonde 2 suit le point focal des faisceaux d'ultrasons Fu. La zone d'intensité maximale du faisceau d'ultrasons Fu est toujours représentée sur l'image de l'écran 22. Pour cela l'échographe 21 peut être couplé à l'ordinateur 15 du transducteur comme on peut le voir sur la figure 1. A la place d'une imagerie échographique, on peut utiliser une imagerie IRM, RX ou un scanner.

Le dispositif de repérage de l'invention comprend également des moyens de monitoring 3 pour détecter une réponse du nerf lorsqu'il est soumis à une stimulation. La réponse du nerf peut être détectée directement au niveau du nerf sous la forme d'une impulsion électrique de réponse ou encore au niveau d'un muscle que le nerf commande. Dans ce dernier cas, la réponse du muscle peut se présenter sous la forme de l'activité électrique du muscle, d'une activité détectable visuellement ou encore d'un changement de pression ou une force. Ainsi, on peut utiliser en tant que moyens de monitoring des appareils du type endoscope, électromyographe, jauge de pression, etc. La jauge de pression peut par exemple être placée sur le muscle cricorayténoïde postérieur. A vrai dire, le type de moyen de monitoring n'est pas un élément critique pour la présente invention, il suffit qu'il puisse détecter une réponse du nerf soumis à une stimulation, que cette stimulation soit électrique, acoustique ou autre.

Le dispositif de repérage comprend également des moyens de stimulation qui peuvent être de forme électrique. Sur la figure 1, le bloc 4 représente des moyens de stimulation électriques comprenant une électrode d'excitation ou de stimulation 41 destinée à être placée de manière à exciter le nerf recherché ou un autre nerf connecté au nerf recherché. Ceci est par exemple le cas sur la figure 2 sur laquelle on peut voir un nerf N₁, en l'occurrence le nerf vagal, et un nerf N₂, en l'occurrence le nerf récurrent laryngé. L'électrode d'excitation reliée au stimulateur électrique 4 est placée de telle manière à pouvoir exciter le nerf vagal N₁ qui va exciter le nerf récurrent laryngé N₂. Le transducteur 1 avec sa sonde échographique associée 2 est appliqué contre la peau du cou de manière à insonifier les tissus du cou et ainsi envoyer les faisceaux d'ultrasons Fu sur le nerf récurrent laryngé N₂. A la place de la stimulation électrique, on peut aussi stimuler le nerf à l'aide de faisceau d'ultrasons. On peut donc se servir du transducteur 1 pour stimuler le nerf. La réponse du nerf peut être surveillée par monitoring au niveau du muscle commandé par le nerf stimulé.

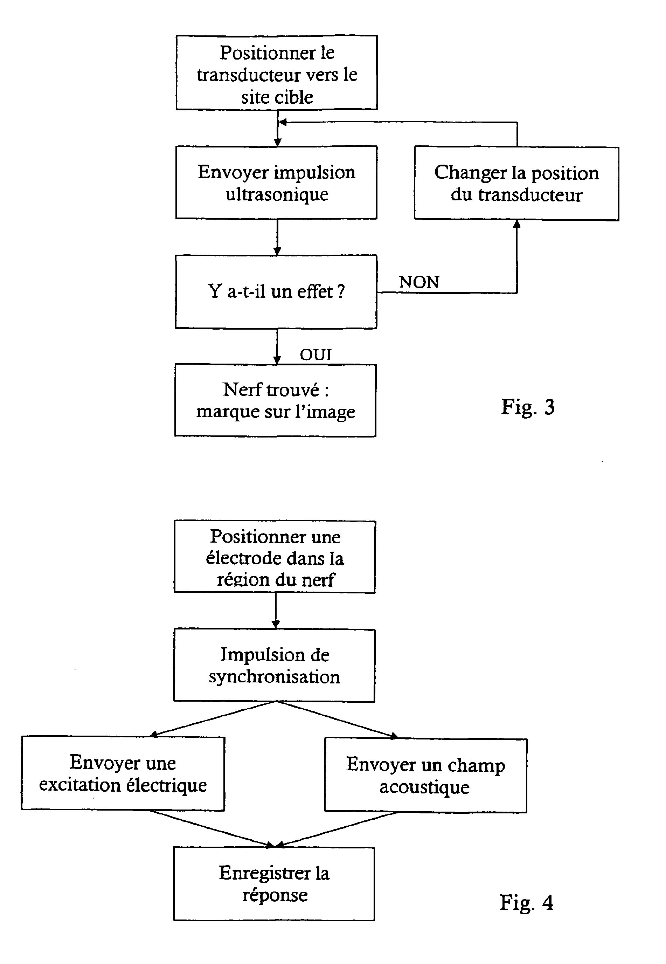

Il est déjà connu dans l'art antérieur qu'un faisceau acoustique suscite perturbe, diminue, augmente ou annule la transmission électrique à l'intérieur des nerfs. Cette capacité des faisceaux acoustiques est ici mise à contribution pour repérer la position d'un nerf. Le principe de l'invention repose sur le fait qu'une réponse perturbée ou induite du nerf est produite lorsque le faisceau d'ultrasons est positionné sur le nerf. Lorsque le nerf est stimulé à l'aide d'impulsions électriques générées par exemple par une technique transcutanée, tel que le Nerve Stimulator Multi Liner de la société TOENIES, il est possible de faire un monitoring de la réponse du nerf ou encore la réponse du muscle auquel le nerf est relié. La réponse détectée par le monitoring va être perturbée par le faisceau d'ultrasons issu du transducteur lorsque celui-ci est positionné sur le nerf. Le transducteur doit donc être déplacé dans la zone du corps où est situé le nerf jusqu'à ce qu'une perturbation de la réponse soit détectée sur les moyens de monitoring. Le transducteur est ainsi déplacé jusqu'à ce que l'on détecte ce changement dans la réponse du nerf. La figure 3 schématise de manière analytique les différentes étapes du procédé de repérage de l'invention. On positionne le transducteur vers la cible, on envoie l'impulsion ultrasonique, on observe s'il y a un effet sur la réponse du nerf. S'il n'y a aucun effet, on change la position du transducteur et on reprend la manipulation. S'il y a un effet, cela signifie que le faisceau est positionné sur le nerf et il faut alors marquer cette position sur l'image visualisée au niveau de l'écran de l'appareil d'imagerie.

Lorsque la stimulation est réalisée à l'aide du transducteur ultrasonore, on peut faire un monitoring de la réponse du nerf au niveau du muscle. Si le muscle réagit, cela signifie à nouveau que le faisceau d'ultrasons est positionné sur le nerf. La réaction du muscle peut être monitorée à l'aide d'un électromyographe, d'un endoscope ou encode d'un capteur de pression.

Il est également possible d'exciter le nerf par le cerveau même du patient. On peut par exemple demander au patient d'effectuer une action et constater si cette action peut toujours être effectuée pendant ou juste après l'insonification. Par exemple, si on veut repérer les nerfs laryngés récurrents, on demandera au patient d'émettre un son, par exemple monotonal et on enregistrera soit les modifications du son de la voix soit les modifications des caractéristiques de l'influx nerveux arrivant sur les muscles par les méthodes décrites ci-dessus.

Le faisceau d'ultrasons influx issu du transducteur 1 ne doit bien entendu pas être à même de détruire le nerf où les tissus environnants dans la zone de recherche. On ne dépassera pas des densités d'énergie (calculées comme le produit de l'intensité ultrasonore par la durée des impulsions) de 1000 watts par cm² x 1000 millisecondes. De préférence, on ne dépassera pas un 10^{ème} de cette valeur. On pourra aussi utiliser des puissances ou intensités ultrasonores fortes mais en impulsion unique (Single Pulse), par exemple proche de celle utilisée pour la lithotritie extracorporelle des calculs rénaux : typiquement, la puissance du faisceau acoustique sera située entre 100 watts par cm² et 100 000 watts par cm² et la durée de l'impulsion sera située entre 1 microseconde et 1 milliiseconde, la pression maximale étant de 1 à 500 bars. Les fréquences ultrasonores seront de quelques MHz, entre 0,5 et 10 MHz, et plus précisément entre 2 et 4 MHz. Les impulsions ultrasonores peuvent être simples ou multiples, longues ou courtes.

Le dispositif de repérage de l'invention comprend également des moyens de synchronisation 5 couplés à la fois au transducteur 1 et aux moyens de stimulation 4 pour synchroniser le faisceau d'ultrasons Fu et l'impulsion électrique de manière à ce qu'ils parcourent le nerf en même temps. Cela permet d'optimiser l'effet du faisceau d'ultrasons sur la réponse électrique du nerf. La synchronisation peut prendre en compte les temps de propagation à la fois de l'influx nerveux et de l'onde ultrasonore dans le tissu afin que les impulsions soient globalement synchrones sur le site d'insonification, c'est-à-dire le nerf, et que l'effet de détection soit maximum. Dans la pratique, les moyens de synchronisation sont reliés à l'alimentation de puissance 13 du transducteur. Selon l'invention, les moyens de synchronisation comprennent également des moyens de décalage 51 adaptés à générer un décalage dans le temps entre le faisceau d'ultrasons et l'excitation électrique de sorte que le faisceau d'ultrasons arrive sur le nerf avant l'impulsion électrique. Un délai de 5 à 20 millisecondes est optimum pour diminuer ou augmenter la transmission de l'influx nerveux, de préférence 7 millisecondes. Ainsi, l'impulsion ultrasonore arrivera sur le nerf 7 millisecondes avant l'impulsion nerveuse, de manière à assurer que l'influx nerveux sera perturbé par l'impulsion ultrasonore. En pratique, les moyens de synchronisation délivrent une impulsion de synchronisation qui déclenche à la fois l'excitation électrique et le transducteur. La réponse du muscle peut alors être enregistrée. La figure 4 représente de manière schématique les différentes étapes de synchronisation mises en oeuvre par la méthode de repérage de l'invention. On commence par positionner l'électrode d'excitation sur la région du nerf, on émet ensuite l'impulsion de synchronisation, l'excitation électrique et le faisceau d'ultrasons sont envoyés et la réponse est détectée.

Il est également possible à l'aide du dispositif de la méthode de repérage de l'invention de déterminer la profondeur du nerf, c'est-à-dire sa position dans la direction de propagation acoustique. En effet, le dispositif comprend des moyens de calcul permettant de déterminer la position en profondeur du nerf à partir du temps de vol acoustique du faisceau d'ultrasons et de la vitesse de propagation de l'influx nerveux.

Dans tous les cas, on excitera point par point la zone d'intérêt avec le faisceau d'ultrasons, en le déplaçant entre les tirs ou les séries de tirs. Lorsque la zone de concentration du faisceau (point focal) atteint le nerf, on obtient une réponse, tel que décrite plus haut. On notera alors la position du point focal, par exemple sur l'image échographique. On aura ainsi repéré le nerf à préserver sur les images échographiques de la région à traiter.

Il est à noter que le dispositif et la méthode de repérage de l'invention utilisent un transducteur ultrasonore ainsi qu'une sonde échographique qui peuvent ensuite être mis en oeuvre pour un traitement thérapeutique ultérieur des tissus avoisinants le nerf. Les seuls éléments additionnels sont alors les moyens de monitoring et les moyens de stimulation.

## Revendications

1. Dispositif de repérage non invasive d'une structure (N₂), telle qu'un nerf, dans une zone d'un corps ayant une surface externe (Se), le dispositif comprenant:
- un transducteur ultrasonore focalisé (1), avantageusement du type HIFU, adapté à produire un faisceau d'ultrasons (Fu) à travers la surface externe (Se) dans la zone, le faisceau (Fu) ayant un point focal capable d'être positionné sur la structure (N₂), et étant capable de perturber la structure (N₃),
- des moyens de monitoring (3) pour détecter une réponse de la structure lorsque soumise à une stimulation, et
- des moyens de stimulation (4) pour stimuler la structure (N₂) qui délivre une réponse détectée par les moyens de monitoring (3), les moyens de stimulation comprennent des moyens de stimulation électrique, **caractérisé en ce que** le dispositif comprend :
- un appareil d'imagerie (2, 21, 22) couplé mécaniquement au transducteur ultrasonore (1) de sorte que l'appareil d'imagerie suit le point focal du transducteur pour visualiser sur une image la position du point focal du faisceau d'ultrasons (Fu) dans la zone de corps,
- des moyens de marquage pour marquer la position de la structure (N₂) sur l'image produite par l'appareil d'imagerie, et
- des moyens de synchronisation (5) pour synchroniser le faisceau d'ultrasons (Fu) et la stimulation de la structure (N₂) par les moyens de stimulation (4) de manière à ce qu'ils parcourent la structure (N₂) en même temps ou avec un décalage temporel déterminé.

2. Dispositif de repérage selon la revendication 1, dans lequel les moyens de stimulation (4) comprennent au moins une électrode (41) capable de délivrer une impulsion électrique à ladite structure (N₂).

3. Dispositif de repérage selon la revendication 2, dans lequel les moyens de synchronisation (5) comprennent des moyens de décalage (51) adaptés à générer un décalage dans le temps entre le faisceau d'ultrasons (Fu) et la stimulation de sorte que le faisceau d'ultrasons arrive sur la structure avant la stimulation.

4. Dispositif de repérage selon l'une quelconque des revendications précédentes, comprenant des moyens de calcul (15) pour déterminer la profondeur de la structure (N₂) à partir du temps de vol acoustique du faisceau d'ultrasons (Fu)dans la zone et la vitesse de propagation de la stimulation.

5. Dispositif de repérage selon l'une quelconque des revendications précédentes, dans lequel les moyens de monitoring (3) comprennent un appareil de monitoring de réponse nerveuse ou un appareil de monitoring de réponse musculaire, tel qu'un électromyographe, ou un capteur de pression.

6. Méthode de repérage non invasive d'une structure (N₂), telle qu'un nerf, dans une zone d'un corps ayant une surface externe (Se), **caractérisée en ce qu'**elle comprend d'insonifier la structure à partir de la surface externe, en délivrant un faisceau d'ultrasons à ladite structure pour la perturber, de faire un monitoring de la réponse de la structure à de stimuler la structure avec des impulsions électriques cette stimulation avec des impulsions électrique et de visualiser la position de l'insonification de la structure, le monitoring étant effectué avec un électromyographe, une jauge de pression ou une détection visuelle d'une activité d'un muscle.

7. Méthode selon la revendication 6, comprenant de synchroniser l'insonification et la stimulation au niveau de la structure.

8. Méthode selon l'une quelconque des revendication 6 ou 7, comprenant de déterminer la profondeur de la structure depuis la surface extérieure à partir du temps de vol acoustique du faisceau d'ultrasons dans la zone et la vitesse de propagation de la stimulation.

## Patentansprüche

1. Nicht invasive Vorrichtung zur Lokalisierung einer Struktur (N₂), wie beispielsweise eines Nervs, in einer Zone eines Körpers mit einer Außenfläche (Se), wobei die Vorrichtung Folgendes umfasst:
- einen fokussierten Ultraschallwandler (1), vorzugsweise vom Typ HIFU, der dazu vorgesehen ist, ein Ultraschallbündel (Fu) durch die Außenfläche (Se) in der Zone zu erzeugen, wobei das Bündel (Fu) einen Brennpunkt hat, der geeignet ist, auf der Struktur (N₂) positioniert zu werden und die Struktur (N₂) zu stören,
- Monitoring-Mittel (3), um eine Antwort der Struktur, wenn sie einer Stimulierung unterzogen wird, zu erfassen, und
- Stimulierungsmittel (4), um die Struktur (N₂) zu stimulieren, die eine Antwort liefert, die von den Monitoring-Mitteln (3) erfasst wird, wobei die Stimulierungsmittel elektrische Stimulierungsmittel umfassen, **dadurch gekennzeichnet, dass** die Vorrichtung Folgendes umfasst:
- ein Bildgebungsgerät (2, 21, 22), das mechanisch mit dem Ultraschallwandler (1) gekoppelt ist, so dass das Bildgebungsgerät dem Brennpunkt des Wandlers folgt, um auf einem Bild die Position des Brennpunktes des Ultraschallbündels (Fu) in der Körperzone sichtbar zu machen,
- Markierungsmittel, um die Position der Struktur (N₂) auf dem vom Bildgebungsgerät erzeugten Bild zu markieren, und
- Synchronisationsmittel (5), um das Ultraschallbündel (Fu) und die Stimulierung der Struktur (N₂) durch die Stimulierungsmittel (4) zu synchronisieren, damit sie die Struktur (N₂) gleichzeitig oder mit einer bestimmten Zeitverzögerung durchlaufen.

2. Lokalisierungsvorrichtung nach Anspruch 2, bei der die Stimulierungsmittel (4) mindestens eine Elektrode (41) umfassen, die geeignet ist, einen elektrischen Impuls an die Struktur (N₂) zu liefern.

3. Lokalisierungsvorrichtung nach Anspruch 1, bei der die Synchronisierungsmittel (5) Verzögerungsmittel (51) umfassen, die geeignet sind, eine Zeitverzögerung zwischen dem Ultraschallbündel (Fu) und der Stimulierung zu erzeugen, so dass das Ultraschallbündel an der Struktur vor der Stimulierung ankommt.

4. Lokalisierungsvorrichtung nach einem der vorhergehenden Ansprüche, umfassend Berechnungsmittel (15), um die Tiefe der Struktur (N₂) auf Basis der akustischen Flugzeit des Ultraschallbündels (Fu) in der Zone und der Ausbreitungsgeschwindigkeit der Stimulierung zu bestimmen.

5. Lokalisierungsvorrichtung nach einem der vorhergehenden Ansprüche, bei der die Monitoring-Mittel (3) ein Monitoring-Gerät für die Nervenantwort oder ein Monitoring-Gerät für die Muskelantwort, wie einen Elektromyographen oder einen Druckfühler, umfassen.

6. Nicht invasive Methode zur Lokalisierung einer Struktur (N₂), wie eines Nervs, in einer Zone eines Körpers mit einer Außenfläche (Se), **dadurch gekennzeichnet, dass** es das Einschallen der Struktur von der Außenfläche aus, wobei ein Ultraschallbündel an die Struktur geliefert wird, um sie zu stören, das Stimulieren der Struktur mit elektrischen Impulsen, ein Monitoring der Antwort der Struktur auf diese Stimulierung mit elektrischen Impulsen und die Anzeige der Position des Einschallens der Struktur umfasst, wobei das Monitoring mit einem Elektromyographen, einem Druckmesser oder einer visuellen Erfassung einer Aktivität eines Muskels erfolgt.

7. Methode nach Anspruch 6, umfassend die Synchronisation des Einschallens und der Stimulierung im Bereich der Struktur.

8. Methode nach einem der Ansprüche 6 oder 7, umfassend die Bestimmung der Tiefe der Struktur von der Außenfläche aus auf Basis der akustischen Flugzeit des Ultraschallbündels in der Zone und der Ausbreitungsgeschwindigkeit der Stimulierung.

## Claims

1. Device for noninvasively locating a structure (N₂), such as a nerve, in a zone of a body having an external surface (Se), the device comprising:
- a focused ultrasonic transducer (1) advantageously of the HIFU type, adapted to produce an ultrasound beam (Fu) through the external surface (Se) into the zone, the beam (Fu) having a focal point which can be positioned on the structure (N₂), and being capable of perturbing the structure (N₂),
- monitoring means (3) for detecting a response of the structure when subjected to a stimulation, and
- stimulation means (4) for stimulating the structure (N₂) which delivers a response detected by the monitoring means (3), the stimulation means comprising electrical stimulation means, **characterized in that** the device comprises
- an imaging apparatus (2, 21, 22) coupled mechanically to the ultrasonic transducer (1) so that the imaging apparatus follows the focal point of the transducer in order to visualize on an image the position of the focal point of the ultrasound beam (Fu) in the body zone,
- marking means for marking the position of the structure (N₂) on the image produced by the imaging apparatus, and
- synchronization means (5) for synchronizing the ultrasound beam (Fu) and the stimulation of the structure (N₂) by the stimulation means (4) so that they travel through the structure (N₂) at the same time or with a determined time offset.

2. Locating device according to Claim 1, in which the stimulation means (4) comprise at least one electrode (41) which can deliver an electrical pulse to said structure (N₂).

3. Locating device according to Claim 2, in which the synchronization means (5) comprise offset means (51) adapted to generate an offset in time between the ultrasound beam (Fu) and the stimulation, so that the ultrasound beam arrives at the structure before the stimulation.

4. Locating device according to any one of the preceding claims, comprising calculation means (15) for determining the depth of the structure (N₂) on the basis of the acoustic time of flight of the ultrasound beam (Fu) in the zone and the propagation speed of the stimulation.

5. Locating device according to any one of the preceding claims, in which the monitoring means (3) comprise an apparatus for monitoring nerve response or an apparatus for monitoring muscle response, such as an electromyograph or a pressure sensor.

6. Method for noninvasively locating a structure (N₂), such as a nerve, in a zone of a body having an external surface (Se), **characterized in that** it comprises insonifying the structure from the external surface, by delivering an ultrasound beam to said structure in order to perturb it, stimulating the structure with electrical pulses, monitoring the response of the structure to this stimulation with electrical pulses, and visualizing the position of the insonification of the structure, the monitoring being carried out with an electromyograph, a pressure gauge or by visual detection of an activity of a muscle.

7. Method according to Claim 6, comprising synchronization of the insonification and the stimulation at the structure.

8. Method according to either one of Claims 6 and 7, comprising determination of the structure from the external surface on the basis of the acoustic time of flight of the ultrasound beam in the zone and the propagation speed of the stimulation.
